# EUROPEAN PATENT SPECIFICATION

(11) **EP 2 050 813 B1**
(45) Date of publication and mention of the grant of the patent: **01.02.2012**
(21) Application number: 08171207.7
(22) Date of filing: 30.03.2006
(51) Int. Cl.: C12N 1/14, C12N 1/20, A01N 63/04

(54) **Use of microbiological consortia of rhizosphere for mycotoxins reduction, and increase in protein and antioxidant compounds contained in agricultural products**
Verwendung von mikrobiologischen Konsortien aus Rhizosphäre zur Mykotoxinreduktion, Eiweiss und Antioxidantienverbindungszunahme in landwirtschaftlichen Produkten
Utilisation de consortiums microbiologiques de rhizosphère pour la réduction de mycotoxines, et l'augmentation des protéines et composés anti-oxydants contenus dans les produits agricoles

(43) Date of publication of application: 22.04.2009
(62) Divisional of application: 06780237.1
(73) Proprietor: Ccs Aosta S.r.l., 10144 Torino (IT)
(72) Inventor: Giovannetti, Giusto, 10123, Torino (IT)
(74) Representative: Freyria Fava, Cristina

(56) References cited:
- EP-A1- 1 074 611
- EP-A1- 1 114 680
- EP-A2- 1 352 694
- WO-A1-2005/105979
- US-A- 5 919 696
- US-A1- 2003 032 170
- US-B1- 6 699 707
- MICKLER C JAN ET AL: "Evaluation of selected geocarposphere bacteria for biological control of Aspergillus flavus in peanut" PLANT AND SOIL, KLUWER ACADEMIC PUBLISHERS, vol. 175, no. 2, 1 August 1995 (1995-08-01), pages 291-299, XP009113403 ISSN: 0032-079X
- DORNER JOE W ET AL: "Aflatoxin reduction in corn through field application of competitive fungi" JOURNAL OF FOOD PROTECTION, DES MOINES, IO, US, vol. 62, no. 6, 1 June 1999 (1999-06-01), pages 650-656, XP009113405 ISSN: 0362-028X
- MARIN SONIA ET AL: "Colonization of maize grain by Fusarium moniliforme and Fusarium proliferatum in the presence of competing fungi and their impact on fumonisin production" JOURNAL OF FOOD PROTECTION, DES MOINES, IO, US, vol. 61, no. 11, 1 November 1998 (1998-11-01), pages 1489-1496, XP009113406 ISSN: 0362-028X
- ANJAIAH V ET AL: "Evaluation of bacteria and Trichoderma for biocontrol of pre-harvest seed infection by Aspergillus flavus in groundnut" BIOCONTROL SCIENCE AND TECHNOLOGY, ABINGDON, GB, vol. 16, no. 4, 1 February 2006 (2006-02-01), pages 431-436, XP009113387 ISSN: 0958-3157
- RODRIGUEZ SB, MAHONEY NE: "Inhibition of aflatoxin production by surfactants" APPL. ENVIRON. MICROBIOL., vol. 60, no. 1, January 1994 (1994-01), pages 106-110, XP002518821
- KABAK BULENT ET AL: "Strategies to prevent mycotoxin contamination of food and animal feed: A review" CRITICAL REVIEWS IN FOOD SCIENCE AND NUTRITION, BOCA RATON, FL, US, vol. 46, no. 8, 1 December 2006 (2006-12-01), pages 593-619, XP009113388 ISSN: 1040-8398
- CARAVACA F ET AL: "EFFECT OF MYCORRHIZAL INOCULATION ON NUTRIENT ACQUISITION, GAS EXCHANGE, AND NITRATE REDUCTASE ACTIVITY OF TWO MEDITERRANEAN-AUTOCHTHONOUS SHRUB SPECIES UNDER DROUGHT STRESS", JOURNAL OF PLANT NUTRITION, MARCEL DEKKER, NEW YORK, NY, US LNKD- DOI:10.1081/PLN-120027547, vol. 27, no. 1, 1 January 2004 (2004-01-01), pages 57-74, XP009085112, ISSN: 0190-4167

## Description

The present invention relates to the use of microbiological consortia of the rhizosphere for obtaining in combination the elimination of nitrate pollutants from agricultural products, the reduction of mycotoxins, as well as the increase of antioxidants, and proteins in the agricultural products themselves. In particular, the knowledge acquired by the present applicant on the use of microbiological consortia of the root as fertilizers has led the present applicant to verify that the use of microbiological consortia of the root determines an improvement in the wholesomeness of foodstuffs through a reduction in carcinogenic substances and mycotoxins, an increase in antioxidant and aromatic substances and a reduction in the amount of nitrates in the agricultural products themselves.

### Technical background of the invention

In order to absorb from the soil the nutritive substances necessary for life, plants need micro-organisms of the rhizosphere that co-operate with the radical apparatus of the plants. It is to be recalled, in fact, that plants evolved from marine algae without roots and when they colonized the land had to establish with some micro-organisms, and more specifically with symbiont fungi, bacteria, actinomycetes and saprophytic fungi, a system of co-operation in order to be able to colonize inhospitable lands.

In particular, the roots of plants establish symbiotic unions with mycorrhizal fungi, which assist the roots in their function of absorbing nutritive substances and water from the soil and receive from the plants the sugars necessary for their life. They amplify the explorative capacity of the root by approximately 600/800 times. In addition, they multiply the normal extension of the radical apparatus; for example, a plant of wheat passes from a length of its root of approximately 200 kilometres to 120 000 or 160 000 kilometres when in symbiosis with mycorrhizal fungi.

The root moreover establishes a relationship of co-operation with bacteria, actinomycetes and saprophytic fungi, which perform for the plant metabolization of the nutritive substances and live off the root exudate produced thereby. It has been calculated that approximately 20% of the substances produced by the plant through chlorophyll photosynthesis is vehicled in the root and used for nourishing the microbiological consortium thereof.

Given in Table 1 are the data regarding the amount of micro-organisms present in the soil.

**Table 1**

| **Micro-organisms** | **Number per gram** | **Biomass (kg/ha,as such)** |
|---|---|---|
| Bacteria | 10⁸-10⁹ | 300-3000 |
| Actinomycetes | 10⁷-10⁸ | 300-3000 |
| Fungi | 10⁵-10⁶ | 500-5000 |
| Micro-algae | 10⁹-10¹⁰ | 10-1500 |
| Protozoa | 10⁹-10¹⁰ | 5-200 |
| Nematodes | 10⁶-10⁷ | 1-100 |

In the last few years, there has been a growing interest in products derived from mycorrhized plants, used mostly as bio-stimulants for improving the growth of the plants and increasing their production yield, for instance in Caravaca et al, J. Plant Nutr. 2004; 27 (1): 57-74, and Mickler et al, Plant and Soil 1995; 175: 291-299..

### Description of the invention

The purpose of the present invention, which is defined by the claims, is to improve the conditions of growth and development of plants for human and animal alimentation and, in particular, to identify natural products that will enable reduction of the amount of harmful (for example, carcinogenic) substances, wherein said harmful substances are mycotoxins, and of nitrates accumulated in plants and, at the same time, an increase of antioxidant substances and of proteins useful to human beings and animals.

A purpose of the present invention is to improve the conditions of growth and development of plants for human and animal alimentation and, in particular, to enable reduction of contamination by fungi that produce mycotoxins, principally in cereal crops.

A further purpose of the present invention is to identify a product that will enable reduction, and possibly complete elimination, of pollutant, in particular carcinogenic, substances from purified waters and, in particular, from waters that undergo phytodepuration.

According to the present invention, the above purpose is achieved thanks to the solution referred to specifically in the ensuing claims. The claims form an integral part of the technical teaching provided herein in relation to the invention.

In a currently preferred embodiment, the present invention relates to the use of a consortium of micro-organisms of the rhizosphere that, if applied to the soil to be cultivated, enables reduction in the amount of pollutants absorbed by or accumulated in cultivated plants and at the same time an increase in the content of antioxidant substances present in the plants themselves.

In the context of cereal crops, but not only, the use of a microbiological consortium of the rhizosphere has enabled elimination of contamination by mycotoxins.

### Detailed description of the invention

The invention will now be described in detail in relation to some currently preferred embodiments, which are provided purely by way of non-limiting example with reference to the annexed figures and in which:
- Figure 1 is a graph of the measurement of PCBs in the specimens identified in Table 4;
- Figure 2 is a graph that represents the antioxidizing capacity of untreated wine and wine treated with micro-organisms of the rhizosphere;
- Figure 3 is a graph that represents the antioxidizing capacity of extracts of untreated peas and peas treated with micro-organisms of the rhizosphere;
- Figure 4 is a graph representing the content of nitrates in green tobacco leaves;
- Figure 5 is a graph representing the content of organic nitrogen in green tobacco leaves; and
- Figure 6 is a graph representing the content of nicotine in dried tobacco leaves.

The present applicant has discovered that it is possible to obtain a reduction of the carcinogenic substances present in the food chain by favouring the metabolization of the substances themselves (for example, polychlorobiphenyls (PCBs), dioxins, insecticides, phytopharmaceutical substances, aromatic polycyclic hydrocarbons, metals, nitrates, etc.) before these are absorbed by plants grown for both human and animal alimentation.

Said filter effect is obtained using as probiotic of the plants the microbiological consortium of the rhizosphere.

In general, in plants as in animals a xenobiotic is biotransformed by enzymes of Phase I and Phase II. In plants, however, the conjugated polar metabolites are not readily excreted, but are further biotransformed, compartmentalized, covalently bonded and/or incorporated as insoluble residue within the plant itself (Phase III). Once assumed by animals or by human beings in the diet, this insoluble residue is subject to other processes of biotransformation, with the consequent release *in vivo* of reactive metabolites and mutagens.

The present applicant has surprisingly discovered that the use of mycorrhizae and of bacteria of the rhizosphere reduces or decreases sensibly the passage of pollutants into plants.

Recent studies conducted by the present applicant have highlighted this innovative aspect of the application of microbiological consortia of the root. Said consortia possess, in fact, micro-organisms that - since they possess an enzymatic complex capable of metabolizing different substances present in the soil - are able to destroy carcinogenic substances, which are consequently not absorbed by the plant. This work is principally performed by the cytochrome P450, which is able to metabolize also substances that are harmful on account of their toxicity and their mutagenic, and consequently carcinogenic, capacity on animal organisms.

### Composition of the microbiological consortium of the rhizosphere

The microbiological consortium of the rhizosphere is basically constituted by mycorrhizae, bacteria, actinomycetes, saprophytic fungi and micromycetes.

Mycorrhizae are symbiotic associations that are set up between the root of many plants and fungi of the subsoil (mycorrhizal fungi). It is believed that they were fundamental in the process of colonization of the continents, and, even today, are still necessary for contemporary vegetation. However, in anthropized environments, such as cultivated land, the mycorrhizae are frequently absent, or else present in a very reduced amount, probably on account of chemical pollution of the soils.

Mycorrhizae are divided into two major groups: ectomycorrhizae and endomycorrhizae.

Ectomycorrhizae are able to colonize few species of plants, almost all forest species (conifers and broadleaf trees) but are of little importance for agricultural crops. They are so defined because they do not penetrate within the tissues, but form a thick layer of mycelium (mantle) around the roots. The known species of ectomycorrhizae number approximately 5 000. They generate spores for their survival and diffusion, which are transported by the wind, by animals, or by the action of human beings. Truffles are the best known expression of this mycorrhizal symbiosis.

Endomycorrhizae are obligate symbionts. Unlike the former, they penetrate within the tissues and cells of the host, but do not form an external fungal mantle. They install on the cortical part of the root, penetrating the cells and filling the intercellular spaces thereof, without, however, ever invading the central cylinder. Within the cells ovoidal structures, referred to as vesicles, and branched structures, referred to as arbuscules, may form. Externally the mycelium can expand around the root up to a few centimetres.

The endomycorrhizae can be divided into five subgroups. In the most common type (vescicular-arbuscular mycorrhizae - VAMs), the fungus, which grows in the soil, penetrates into the root cells, where it forms branched structures (arbuscules). It is in the arbuscules that the nutritional exchanges occur: the fungus absorbs the nutritive elements from the soil, in particular phosphorous, potassium and some micro-elements, and yields them to the plant in order to receive therefrom in exchange processed lymph. The formation of mycorrhizae bestows upon the plant a greater capacity for absorption of the water and protection from attack by certain root pathogens. The sum of these effects guarantees a better growth in mycorrhized plants.

Mycorrhizae are able to solubilize and hence to absorb the organic or mineral forms present in the soil in the form of insoluble compounds, which are not directly usable by the plants, changing radically the agronomic count of the availability of the nutritive elements in the soil. The greater absorption of mineral salts from the soil (P, N, Ca, K, Fe, Mg, Cl, Zn, Cu) modifies the equilibrium and the composition of the nutrients in the plant tissues, with consequences on the photosynthetic yield, which increases, and on sharing of the products of photosynthesis between the root and the aerial part of the plant. Of the total carbon assimilated by the plant 20% can be transferred to the fungus.

The bacteria of the rhizosphere, the actinomycetes, the saprophytic fungi and the micromycetes create an assimilative network capable of extracting the nutrients naturally present in the soil, rendering them available to the plant, and metabolize the carcinogenic substances.

Given in Table 2 are the main components of a particular embodiment of the microbiological consortium of the rhizosphere used in the framework of the present invention.

**Table 2**

| ***Micromycetes*** | ***Saprophytic fungi*** | ***Actinomycetes*** | ***Bacteria of the rhizosphere*** | ***Endomycorrhizal fungi*** |
|---|---|---|---|---|
| *Mortierella* isabellina | *Trichoderma* spp. | *Streptomyces* spp. | *Pseudomonas* spp | *Glomus* spp |
| *Aspergillum* spp | *Trichoderma* harthianum | *Streptomyces* griseus | *Pseudomonas* borealis | *Glomus* coronatum |
| *Aspergillum* niger | *Trichoderma* viridae | *Streptomyces* avermitilis | *Pseudomonas* fluorescens | *Glomus* caledonium |
| *Sclerotium* spp | *Trichoderma* aureoviridae | | *Pseudomonas* synxantha | *Glomus* intreradices |
| *Ulocladium* spp | *Trichoderma* atroviridae | | *Pseudomonas* corrugata | *Glomus* viscosum |
| *Ulocladium* oudemansii | *Trichoderma* koningii | | *Pseudomonas* aeroginosa sp. | *Glomus* mosseae |
| *Arthrobotrys* spp | *Trichoderma* harzianum | | *Pseudomonas* aeroginosa | *Glomus* glomaceae |
| *Arthrobotrys* oligospora | *Trichoderma* virens | | *Bacillus* spp | *Glomus* fasciculatum |
| *Fusarium* spp | *Trichoderma* Hypocrea schweinitzii an. | | *Bacillus* subtilis | *Glomus* claroideum |
| *Mucor* spp. | *Trichoderma* viride | | *Bacillus* megaterium | *Glomus* etunicatum |
| *Pichia* spp | *Coniothyrium* spp | | *Bacillus* polymyxa | *Glomus* epigaeum |
| *Pichia* pastoris | *Coniothyrium* minitans | | *Bacillus* licheniformis | *Glomus* lamellosum |
| | *Gliocadium* spp | | *Alcanivorax* spp | *Glomus* monosporum |
| | *Gliocadium* calenulatum | | *Alcanivorax* venetianus | *Acaulospora* spp |
| | *Beauveria* spp | | *Candida* spp | *Acaulospora* longula |
| | *Beauveria* bassiana | | *Rhodococcus* spp | *Acaulospora* laevis |
| | | | *Rhodococcus* rythropolys | *Gigaspora* spp |
| | | | *Acinetobacter* spp | *Gigaspora* ramisporophora |
| | | | *Acinetobacter* calcoaceticus | *Gigaspora* gigantea |
| | | | *Acinetobacter* Radioresistens | *Gigaspora* margarita |
| | | | *Mycobacterium* spp | *Gigaspora* rosea |
| | | | *Mycobacterium* thermoautotrophium | *Gigaspora* calospora |
| | | | *Agrobacterium* spp | *Scutellospora* spp |
| | | | *Agrobacterium* radiobacter | *Scutellospora* calospora |
| | | | *Serratia* spp | |
| | | | *Serratia* marcescens | |

It is obvious that persons skilled in of the branch will carry out the necessary analyses to verify the possible pathogenicity of the individual strains of the different micro-organisms constituting the microbiological consortium of the rhizosphere to be employed in order to avoid the use of strains that are pathogenic for plants.

The composition of the inoculation of the mycorrhizal consortium of the rhizosphere has the following minimum content of microbiological components:
i) endomycorrhizal fungi are present in a minimum percentage of 6 wt%, preferably 10-20 wt% with respect to the total weight of the product;
ii) bacteria of the rhizosphere are present to a minimum extent of 1x10⁶ CFU/g, preferably 5-10x10⁶ CFU/g, of dry weight with respect to the total weight of the product;
iii) antagonistic saprophytic fungi and micromycetes are present to a minimum extent of 1x10⁶ CFU/g, preferably 5-10x10⁶ CFU/g, of dry weight with respect to the total weight of the product;
iv) actinomycetes are present to a minimum extent of 1x10⁶ CFU/g, preferably 5-10x10⁶ CFU/g, of dry weight with respect to the total weight of the product; and
v) micromycetes are present to a minimum extent of 1x10⁶ CFU/g, preferably 5-10x10⁶ CFU/g, of dry weight with respect to the total weight of the product.

The modalities of application of the microbiological consortium of the rhizosphere can be summarized as described in what follows.

When used for treating the seeds directly, in order to obtain a greater adhesion to the seeds themselves, the consortium can be glued with the usual glues used in the treatment of seeds; in this case, a concentrated product needs to be used in a range of between 200 g and 400 g per hectare dose of seeds to be treated.

For mixing in loam and peat used in seed boxes for the production of young plants and cuttings, the consortium is used in a recommended dose of 15 litres per cubic metre of loam or peat.

When making the distribution at the moment of sowing in field, the consortium is spread directly by the seeders, provided with microgranulators, in a dose equal to approximately 13-20 litres per hectare.

In the case of broadcast sowing in cropping beds, the consortium is distributed directly prior to sowing or to transplantation of the young plants themselves at a dose of approximately 100 l/ha.

In the case of direct distribution in cultivations (e.g. vineyards, fruit orchards, olive groves), the consortium is distributed slightly buried, with a light ploughing, or with an injector tube, at a dose of 100-200 l/ha.

The use of the microbiological consortium of the rhizosphere not only contributes directly to the development of the treated plants, but indirectly favours a series of other processes, such as for example:
- rendering the plants healthier and more resistant to diseases, thus increasing their capacity for root absorption;
- increasing the resistance of plants to attack by nematodes;
- extending the radical apparatus by up to 600-800 times, with consequent considerable increase in its potentiality for absorption of nutritive elements;
- enhancing the perfume, pigmentation, and organoleptic qualities in general of the edible part of the plants;
- rendering available and readily assimilable by the plants macro-elements (N, P, K) and micro-elements present in the soil, including ones not accessible by the radical apparatus alone of the plants themselves;
- enriching the soil with organic biomass, favouring a greater uniformity of the vegetal development and increasing production in the subsequent years.

Various experimental tests highlight the positive results in the application of mycorrhization with the consortia of micro-organisms of the rhizosphere, with benefits both at an environmental level and at an alimentary level.

In what follows, experimental data in relation to the in-field use of the microbiological consortium of the rhizosphere will be provided. Highlighted in what follows are the benefits afforded by the consortium via the so-called "filter effect" performed by the micro-organisms of the rhizosphere in regard to harmful or carcinogenic substances and pesticides present in the soil to prevent these from possibly reaching the end vegetal product and consequently the food chain. There will emerge the effectiveness of the consortium in increasing antioxidants and in reducing the amount of nitrates in the end vegetal product thanks to the capacity of increasing the photosynthetic efficiency of the plants inoculated with the consortium, and likewise there will emerge an increase in the amount of protein of the end vegetal product, such as for example in wheat and maize.

### EXAMPLES

### Example 1. Reduction of carcinogenic substances in the food chain

The present applicant has discovered that the use of mycorrhizae and bacteria of the rhizosphere constitutes a surprisingly advantageous tool for reducing (and, in some cases, even preventing) the passage of chemical pollutants into the food chain thanks to the metabolic work of the cytochromes P450 and of the conjugation enzymes present in the mycorrhizal fungi and in the bacteria of the rhizosphere.

Appearing in Table 3 are data regarding the presence of the enzymes responsible for metabolization of carcinogenic substances in each of the micro-organisms present in the microbiological consortia of the rhizosphere tested by the present applicant.

**Table 3**

| **SPECIFIC ACTIVITY** | **BACILLUS SUBTILIS** | **PSEUDOMONAS FLUORESCENS** | **TRICHODERMA** | **GLIOCLADIUM** |
|---|---|---|---|---|
| **CATALASE ^{a}** | | 23.34+0.01 | 150.45±30.09 | 8+1.6 |
| **DT-DIAPHORASE^{b}** | 114.44±20.24 | 30.15±8.23 | 66.35±15.82 | 25.70±10.45 |
| **ECOD^{c}** | 0.162±0.011 | n.d. | 0.685±0.137 | 0.84±0.61 |
| **GLUTATHIONE PEROXIDASE^{b}** | | 1.77±0.37 | 5.94±2.64 | 2.81±0.81 |
| **GLUTATHIONE REDUCTASE ^{b}** | | 82.08±25.34 | 3.93±0.88 | 17.11±7.22 |
| **GLUTATHIONE S-TRANSFERASE** | n.d. | n.d. | 9.34±1.868 | n.d. |
| **NADPH-CYTOCHROME C REDUCTASE ^{c}** | 26.81±4.95 | 5.24±1.61 | 7.12±3.99 | 3.28±0.92 |
| **SUPEROXIDE DISMUTASE ^{d}** | | 5.50±2.05 | 250.3±26.45 | 23.58±13.13 |

| | | | | |
|---|---|---|---|---|
| ^{a} Specific activity, expressed in µmol/min x mg prot. ^{b} Specific activity, expressed in nmol/min x mg prot. ^{c} Specific activity, expressed in pmol/min x mg prot. ^{d} Specific activity, expressed in U/mg prot. n.d. activity not determinable | | | | |

The experimentation was conducted, evaluating in particular the polychlorinated biphenyls (PCBs) [1-6].

PCBs constitute a group of 209 congeners [2] produced by the chlorination of two benzene groups joined to one another by just one bond; they may be grouped together into 10 families of isomers from mono to deca according to the number of substituent chlorine atoms. The PCBs are, then, characterized by a considerable variability of structure with chlorine atoms in *ortho, meta* and para position, with a marked spatial symmetry or asymmetry that ranges from complete planarity of the two benzene groups up to non-planarity, in which the benzene rings are at 90° with respect to one another. The number and position of the chlorine atoms present condition and govern accumulation and persistence of the different congeners in the different environmental matrices, as a consequence of the different chemical and biological reactivity; the equal variability of the chemical, chemico-physical and toxicological properties thereof do not determine their destiny and dangerousness. The individual congeners are identified by an increasing numbering system, referred to as BZ number, after the name of the drafters of the classification, Ballschmiter and Zell 1980, which follows the IUPAC characterization for the identification of the position of the chlorine atoms. For example, PCB 126 indicates the congener 3,3',4,4',5 penta-CB.

Listed in what follows are the 24 congeners of PCBs taken into consideration for the analysis made in the context of the present patent application; 81 (3,4,4,5'-TeCB); 77 (3,4,3',4'-TeCB); 126 (3,4,5,3',4'-PeCb); 128 (2,3,4,2',3',4'-HxCB); 156 (2,3,4,5,3',4'-HxCB); 169 (3,4,5,3',4',5'-HxCB); 138 (2,3,4,2',4',5'-HxCB); 187 (2,3,5,6,2',4'5'-HpCB); 183 (2,3,4,6,2',4',5'-HpCB); 177 (2,3,5,6,2',3',4'-HpCB); 180 (2,3,4,5,2',4',5-HpCB); 170 (2,3,4,5,2',3',4'-HpCB); 28 (2,4,4'-TeBC) 52(2,5,2',5'-TeCB); 95(2,2',3,5,6-PeBC); 101 (2,4,5,2',5'-PeCB); 99 (2,4,5,2',4'-PeCB); 110 (2,3,3',4',6-PeCB); 151 (2,2',3,5,5',6-HxCB); 149 (2,2',3,4',5,6'-HbCB); 118 (2,4,5,3',4'-PeCB); 146 (2,2',3,4',5',5'-HeCB); 153 (2,4,5,2',4',5'-HxCB); 105 (2,3,4,3',4'-PeCB).

In the experimentation conducted by the present applicant, different measurements were obtained in different agricultural products before and after the application of the consortium of micro-organisms of the rhizosphere. The treatment envisaged application of the consortium and in addition - in some cases - application of biosurfactants (of natural or synthetic origin) in order to increase the availability of liposoluble pollutant compounds. Biosurfactants that are usable to advantage in the framework of the present invention are cyclodextrins and polyethylene sorbitol ester, marketed under the trade name of TWEEN 80.

Given in Table 4 are the data regarding the specimens analysed.

**Table 4**

| **Specimen** | **Description** | **Lot** | **In-field treatment** |
|---|---|---|---|
| 1fM | MAIZE leaves and stems | 1 Y | No in-field treatment |
| 1gm-fruit | MAIZE fruit | 1 Y | |
| 1rM | MAIZE roots | 1 Y | |
| 1tP | POTATO tubers | 1 Z | No in-field treatment |
| 1fP | POTATO leaves | 1 Z | |
| 2fC | CANAPA leaves, stems and fruit | 2 X+Y+Z | Treatments with the consortia of the rhizosphere |
| 2rC | CANAPA root | 2 X+Y+Z | |
| 3fC | CANAPA leaves, stems and fruit | 3 X+Y+Z | In-field treatment with the consortia of the rhizosphere + biosurfactant micro-organisms |
| 3rC | CANAPA roots | 3 X+Y+Z | |
| 4fM | MAIZE leaves and stems | 4 X+Y+Z | Treatments with the consortia of the rhizosphere |
| 4gM-fruit | MAIZE fruit | 4 X+Y+Z | |
| 4rM | MAIZE roots | 4 X+Y+Z | |
| 5fM | MAIZE leaves and stems | 5 X+Y+Z | In-field treatment with the consortia of the rhizosphere + biosurfactant micro-organisms |
| 5gM-fruit | MAIZE fruit | 5 X+Y+Z | |
| 5rM | MAIZE roots | 5 X+Y+Z | |
| 6fP | POTATO leaves | 6 X+Y+Z | Treatments with the consortia of the rhizosphere |
| 6tP | POTATO tubers | 6 X+Y+Z | |
| 7fP | POTATO leaves | 7 X+Y+Z | In-field treatment with the consortia of the rhizosphere + biosurfactant micro-organisms |
| 7tP | POTATO tubers | 7 X+Y+Z | |

The moist material was cut and reduced into small pieces. The seeds were crushed in a steel mortar. The roots and the potatoes were washed with water, and the traces of soil were removed using an ultrasound bath. Approximately 10 g of material for leaves and stems and 30 g for seeds and tubers of the potatoes were weighed, placed in a cellulose extraction thimble and extracted in 80-100 ml of dichloromethane using a Soxhlet (Soxhtech) system.

The extract was purified from organic substances that interfere with concentrated sulphuric acid; the organic phase, separated by centrifuging, was purified with a Florisil column (8-10 g). The dichloromethane was removed with a rotavapor by addition of hexane, and then the specimen was brought down to a small volume (200 µl) in nitrogen flow.

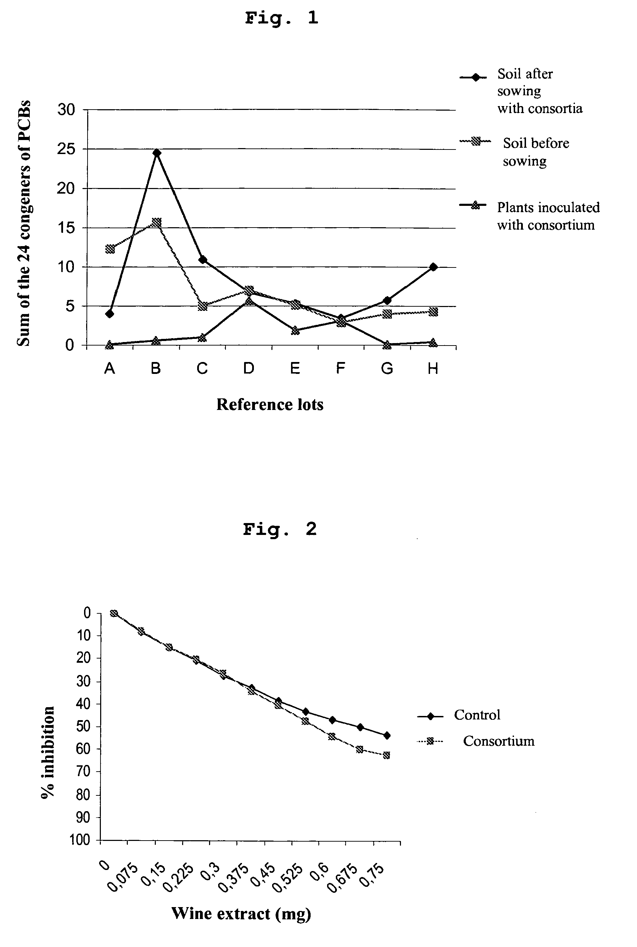

Appearing in Table 5 are the values of the PCBs of the 24 congeners mentioned above, encountered in the different lots; there may be noted a reduction of the concentration of PCBs in the soil sampled and analysed after sowing in the soils treated with the consortia of micro-organisms of the rhizosphere. It is to be noted that in the untreated lots the absorption is almost zero: this means that the untreated soils maintain the same initial contamination of PCBs. Represented graphically in Figure 1 are the values of Table 5.

The comparison was made between the mean of the values of each lot (x+y+z), where by the term "lot" is meant a small plot or enclosure of the total land.

**Table 5**

| **Specimens** | **Soil before sowing** | **Soil after sowing with consortia** | **Plants inoculated with consortium** |
|---|---|---|---|
| A | 4 | 12.3 | 0.1 |
| B | 24.5 | 15.7 | 0.6 |
| C | 10.9 | 5 | 1 |
| D | 6.7 | 7 | 5.7 |
| E | 5.3 | 5.1 | 1.9 |
| F | 3.4 | 2.9 | 3.1 |
| G | 5.7 | 4 | 0.1 |
| H | 10 | 4.3 | 0.4 |

### Example 2. Increase of anti-oxidant substances in the end product

The present applicant has also found that the application of the consortia of micro-organisms of the rhizosphere causes, at a physiological level, a change in the formation of antioxidant substances, in particular, with an increase in the amount of antioxidant substances present in the end agricultural product.

The tests were conducted on wine and peas coming from plants cultivated with consortia of micro-organisms of the rhizosphere.

The anti-oxidizing power of the extracts of wine and peas was evaluated using a spectrophotometric method.

By means of a peroxidase reaction a radical compound was produced that had a maximum of absorption at 730 nm. The addition in the reaction mixture of antioxidant substances causes a reduction in the spectral absorption and, on the basis of said reduction, the antioxidizing capacity of the compound examined was evaluated.

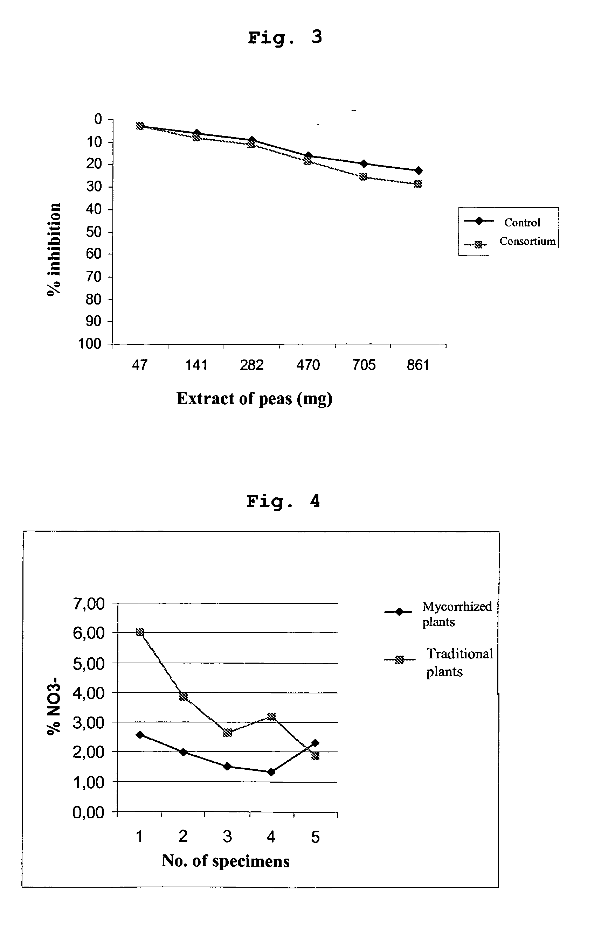

As may be noted from Figures 2 and 3, wine and peas coming from plants cultivated with the consortium of micro-organisms of the rhizosphere present an anti-oxidizing power greater than the ones coming from untreated plants.

### Example 3. Reduction of nitrates in the end product

The use of microbiological consortia of the root moreover has the effect of improving the photosynthetic efficiency, with consequent reduction of the nitrates in the leaf, and that of favouring the absorption of nitrates and nitrites present in the agricultural soil that are not normally accessible for the root.

### A. Experimentation conducted on the tobacco plant

The experimentation on the tobacco plant was conducted using nitrogenous fertilizer in the correct limits in order to conserve and improve the quality of the tobacco product, not to jeopardize the quantitative yields, and to mitigate the impact of the cultivation of tobacco on the environment. As regards the latter point, a particularly serious aspect due to the excessive use of mineral fertilizers is the pollution of waters by nitrates, which in some countries has amounted to a real environmental emergency.

In the present experimentation, the values of the nitrate content of tobacco leaves (Burley and Bright) coming from mycorrhized plants, and from non-mycorrhized (i.e., traditional) plants were compared. The latter, as regards the Burley, following the normal practices of cultivation, envisage the administration of approximately 240 kg/ha of mineral nitrogen (160 kg/ha for bottom fertilization and 80 kg/ha for cover fertilization). For the mycorrhized plants, instead, which envisage the use of 100 l/ha of a microbiological consortium of the rhizosphere and an organic bottom fertilization with approximately 200 q/ha of buffalo manure, the mineral nitrogen is spread only as cover fertilization and reduced to 22 kg/ha i.e., by a factor of approximately 10. Given in Table 6 are the percentage values of nitric and organic nitrogen in green leaves. The results refer to the dry content at 105°C.

**Table 6**

| **MYCORRHIZED PLANTS** | | | | | | | | | | |
|---|---|---|---|---|---|---|---|---|---|---|
| | 1st specimen | | 2nd specimen | | 3rd specimen | | 4th specimen | | 5th specimen | |
| | %NO₃⁻ | %org. N | %NO₃⁻ | %org. N | %NO₃⁻ | %org. | N %NO₃⁻ | %org. | N %NO₃⁻ | %org. N |
| 1st det. | 2.59 | 3.07 | 1.95 | 2.90 | 1.48 | 3.14 | 1.33 | 3.22 | 2.28 | 3.36 |
| 2nd det. | 2.55 | 3.10 | 1.98 | 2.95 | 1.50 | 3.07 | 1.29 | 3.15 | 2.32 | 3.40 |
| 3rd det. | 2.57 | 3.19 | 1.98 | 2.95 | 1.54 | 3.10 | 1.30 | 3.18 | 2.34 | 3.36 |
| **Mean** | **2.57** | **3.12** | **1.97** | **2.93** | **1.51** | **3.10** | **1.31** | **3.18** | **2.31** | **3.37** |
| **S.D.** | 0.02 | 0.06 | 0.02 | 0.03 | 0.03 | 0.04 | 0.02 | 0.04 | 0.03 | 0.02 |
| | | | | | | | | | | |

| **NON-MYCORRHIZED PLANTS** | | | | | | | | | | |
|---|---|---|---|---|---|---|---|---|---|---|
| | 1st specimen | | 2nd specimen | | 3rd specimen | | 4th specimen | | 5th specimen | |
| | %NO₃⁻ | %org. N | % NO₃⁻ | %org. N | %NO₃⁻ | %org. | N %NO₃⁻ | %org. | N %NO₃⁻ | %org. N |
| 1st det. | 6.11 | 4.02 | 3.88 | 4.26 | 2.68 | 3.71 | 3.27 | 4.18 | 1.83 | 2.66 |
| 2nd det. | 5.96 | 3.96 | 3.82 | 4.23 | 2.60 | 3.76 | 3.12 | 4.10 | 1.90 | 2.75 |
| 3rd det. | 5.98 | 4.10 | 3.83 | 4.20 | 2.64 | 3.80 | 3.15 | 4.20 | 1.85 | 2.70 |
| **Mean** | **6.02** | **4.03** | **3.84** | **4.23** | **2.64** | **3.76** | **3.18** | **4.16** | **1.86** | **2.70** |
| **S.D.** | 0.08 | 0.07 | 0.03 | 0.03 | 0.04 | 0.05 | 0.08 | 0.05 | 0.04 | 0.05 |

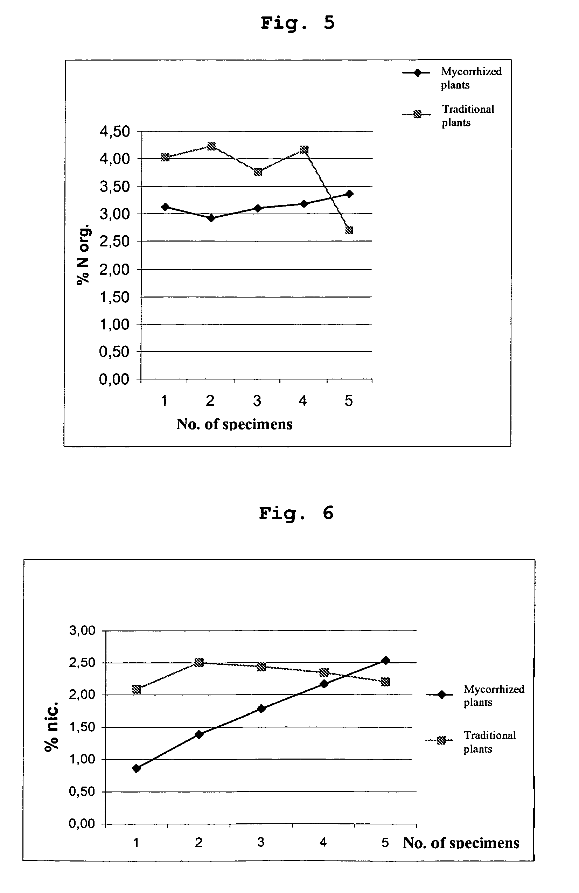

It may be seen clearly that, in the leaves of mycorrhized plants, the percentage of nitrates is in all cases less than in those of non-mycorrhized plants. Also the pattern of the organic nitrogen is similar to that of the nitrates, as emerges clearly from Figures 4 and 5.

The pattern of the values of nicotine (Figure 6) reflects the one regarding nitrates and organic nitrogen in green leaves.

The results obtained by the present applicant through the use of a consortium of micro-organisms of the rhizosphere were positive. In particular, there was recorded a reduction of the chemical product used, with consequent containment of the costs and mitigation of the impact of the cultivation on the environment and a raising of the standards that qualitatively distinguish Burley tobacco.

### B. Experimentation conducted on salad crops

The experimental tests conducted with crops of salad show a significant reduction of the nitrates in the leaf as compared to the fertilized control, and an even more positive result with the reduction of the nitrogenous fertilizer.

### C. Experimentation conducted on potato crops

It is interesting to note, in the results obtained from these analyses (Tables 7 and 8), how the amount of nitrates (mg/kg) decreased in the crops that were treated with the microbiological consortium of the rhizosphere, unlike what occurred in plants that underwent traditional treatment.

**Table 7**

| **Application** | **Wt. of spec. (kg)** | **Nitrates (as such) (mg/kg)** |
|---|---|---|
| Control | 3.4 | 95.8 |
| Product A | 3.5 | 56.6 |
| Product B | 3.5 | 63.6 |

**Table 8**

| **Application** | **Wt. of spec. (kg)** | **Nitrates (as such) (mg/kg)** |
|---|---|---|
| Control | 3.3 | 87.0 |
| Product A | 3.5 | 63.4 |
| Product B | 3.6 | 67.3 |

| | | |
|---|---|---|
| Control: absence of mycorrhization Product A: endomycorrhizal symbiont fungi of the genus Glomus (G. viscosum, G. coronatum), bacteria of the rhizosphere (P. fluorescens, Pseudomonas spp, Bacillus subtilis and Streptomyces spp.) and antagonistic saprophytic fungi (Trichoderma spp.) to a minimum extent of 5x10⁶ C.F.U./g. Product B: endomycorrhizal symbiont fungi of the genus Glomus (G. viscosum, G. coronatum), bacteria of the rhizosphere (Pseudomonas fluorescens, Pseudomonas spp, Bacillus subtilis and Streptomyces spp.) and antagonistic saprophytic fungi (Trichoderma spp.) to a minimum extent of 5x10⁶ C.F.U./g. | | |

### Example 4. Increase in the protein content, gluten, and forage unit in foodstuffs

The present applicant has found a surprising increase in the protein quality of agricultural products following upon the application of a microbiological consortium of the rhizosphere. In what follows, some results are given regarding the increase in gluten in durum wheat, the increase of the forage unit and proteins in shredded maize silage and in alfalfa. It is moreover pointed out that mycorrhization favours the assumption, in a balanced way, of micro-elements that determine the thickening of the cell walls, with consequent increase in weight of the plants and of the fruit.

**Table 9 Mycorrhization of tomatoes**

| **Test** | **Type of test** | **Optical residue °Bx** | **Bostwik cm/30 s.** | **Comm. Product. tonnes/ha** |
|---|---|---|---|---|
| 1 | Without consortium | 4.40 | 8.2 | 97.00 |
| | With consortium | 4.90 | 3.8 | 103.90 |
| | Δ% | + 11.40 | - 53.70 | + 7.10 |
| 2 | Without consortium | 4.60 | | 67.25 |
| | With consortium | 4.95 | Not detected | 78.45 |
| | Δ% | + 7.60 | | + 16.70 |
| 3 | Without consortium | 4.00 | | 72.77 |
| | With consortium | 4.25 | Not detected | 76.97 |
| | Δ% | + 6.30 | | + 5.80 |
| Mean | Without consortium | 4.30 | 8.2 | 79.00 |
| | With consortium | 4.70 | 3.8 | 86.40 |
| | Δ% | + 9.30 | - 53.70 | + 9.40 |

**Table 10 Mycorrhization of maize**

| **Shredded maize** | **Without consortium** | **With consortium** | **Δ%** |
|---|---|---|---|
| Moisture % | 57.10 | 56.10 | - 1.75 |
| Proteins % | 3.10 | 3.15 | - 0.64 |
| Fats % | 0.96 | 0.93 | - 3.13 |
| Fibres % | 9.91 | 8.90 | - 10.19 |
| Ashes % | 1.57 | 1.53 | - 2.55 |
| F.U./q | 34.44 | 38.21 | + 10.95 |
| Nitrates (mg/kg) | 231.27 | 151.79 | - 34.37 |
| Weight of plant (kg) (average over 40 plants) | 0.627 | 0.728 | + 16.11 |

**Table 11 Mycorrhization of maize**

| **Maize grain for pig feed** | **Without consortium** | **With consortium** | **Δ%** |
|---|---|---|---|
| Moisture % | 34.03 | 32.37 | - 4.88 |
| Proteins % | 5.84 | 6.40 | + 9.59 |
| Fats % | 1.99 | 2.58 | + 29.65 |
| Fibres % | 2.15 | 2.57 | + 19.53 |
| Ashes % | 0.96 | 0.96 | ------ |
| Specific weight | 67.57 | 71.67 | + 6.07 |
| Nitrates (mg/kg) | 46.97 | 27.74 | - 40.94 |

**Table 12 Mycorrhization of alfalfa**

| **Alfalfa** | **Without consortium** | **With consortium** | **Δ%** |
|---|---|---|---|
| Moisture % | 84.31 | 79.34 | - 5.89 |
| Proteins % | 4.52 | 5.70 | + 26.11 |
| Fats % | 0.46 | 0.61 | + 32.61 |
| Fibres % | 3.45 | 4.80 | + 39.13 |
| Ashes % | 1.76 | 2.26 | + 28.41 |
| F.U./q | 12.04 | 15.40 | + 27.91 |
| *Nitrates (mg/kg) | 1.095.28 | 936.95 | - 14.45 |

**Table 13 Mycorrhization of durum wheat**

| **Application** | **Replicat.** | **%Moist.** | **Electrolyt. weight** | **Wt. of spec. (grams)** | **Prot.% grain** | **Gluten% grain** | **Weight 1000 seeds** |
|---|---|---|---|---|---|---|---|
| Control | 1 | 12.3 | 81.5 | 450 | 10.1 | 7.6 | 51.5 |
| | 2 | 12. 6 | 82.8 | 375 | 10.2 | 7.5 | 49.0 |
| | 3 | 12.3 | 82.4 | 350 | 10.2 | 7.6 | 51.0 |
| Product A | 1 | 12.3 | 84.6 | 362 | 12.2 | 9.6 | 55.0 |
| | 2 | 12.3 | 84.6 | 372 | 12.2 | 9.7 | 54.0 |
| | 3 | 12.4 | 85.0 | 354 | 12.2 | 9.7 | 55.5 |
| Product B | 1 | 12.3 | 84.0 | 321 | 12.3 | 9.9 | 56.0 |
| | 2 | 12.2 | 84.5 | 360 | 12.4 | 9.9 | 55.5 |
| | 3 | 12.3 | 84.1 | 375 | 12.5 | 10.1 | 54.0 |

| | | | | | | | |
|---|---|---|---|---|---|---|---|
| Control: absence of mycorrhization Product A: endomycorrhizal symbiont fungi of the genus Glomus (G. viscosum, G. coronatum), bacteria of the rhizosphere (P. fluorescens, Pseudomonas spp, Bacillus subtilis and Streptomyces spp.) and antagonistic saprophytic fungi (Trichoderma spp.) to a minimum extent of 5x10⁶ C.F.U./g. Product B: endomycorrhizal symbiont fungi of the genus Glomus (G. viscosum, G. *coronatum),* bacteria of the rhizosphere (Pseudomonas fluorescens, *Pseudomonas* spp, *Bacillus subtilis* and *Streptomyces* spp.) and antagonistic saprophytic fungi (*Trichoderma* spp.) to a minimum extent of 5x10⁶ C.F.U./g. | | | | | | | |

The analyses reveal a clear increase in the total protein percentage of the product harvested, an increase in the percentage of gluten, and an increase in the electrolytic weight on the treated plants as compared to the untreated produce (control).

### Example 5. Barrier to mycotoxins produced by moulds and fungi

The most frequent attacks on cereals by fungi and moulds that produce mycotoxins is represented by the genera *Aspergillus spp., Fusarium spp., Claviceps spp.,* which produce aflatoxins, trichothecenes and ergotamine, respectively.

The presence of mycotoxins in the food chain may mean a risk for human health, where the seriousness of the risk is determined by the amount and type of mycotoxin.
Recent studies of the Bayer company show that these fungi are able to infect the entire plant, penetrating from the roots of the plants themselves, and through the lymphatic channels the infection is transmitted to the entire plant.

Application of the consortia of micro-organisms of the rhizosphere blocks infection by these organisms in so far as fungi belonging to the consortium (for example, *Trichoderma spp.)* function as antagonists, occupying the niches of the roots and preventing the fungi that produce mycotoxins from entering the lymphatic channels of the plant and preventing propagation of infection.

Of course, the details of implementation and the embodiments may vary widely with respect to what is described and illustrated herein, without thereby departing from the sphere of protection of the present invention, as defined in the annexed claims.

### Bibliography

1. ATSDR (Agency for Toxic Substances and Disease Registry), (2000), "Toxicological profile for polychlorinated biphenyls". Atlanta: ATSDR, draft for public comment
2. ENVi-MOD,SMIC(2003) "Considerazioni in merito alle problematiche per la condotta dell'analisi di rischio da PCB" 8th International Conference on Contaminated Soil, Belgium, May 12-16, 2003
3. Institute for Evaluating Health Risks (1991), "Reassessment of liver findings in five PCBs studies rats. Washington D.C; dated July 1. Report submitted to U.S EPA
   IRIS Integrated Risk Information System, US EPA; www.epa.gov/iris
4. National Cancer Institute (1978) "Bioassay of Aroclor 1254 for possible carcinogenicity" Carcinogenesis Tech. Rep Ser. No. 38
5. U.S APA Environmental Protection Agency (1991)" Workshop report on toxicity equivalency factors for polychlorinated-biphenyl congeners" Risk Assessment Forum, Washington D.C., Report No. EPA /625/3-91/020
6. WHO World Health Organization (1993) "Polychlorinated biphenyls and terphenyls" Geneva: WHO, Environmental Health Criteria 140, 2nd edition

## Claims

1. Use of a consortium of micro-organisms of the rhizosphere in the cultivation of plants for human or animal alimentation for obtaining
reduction of harmful substances accumulated in plants, *wherein said harmful substances are mycotoxins,*
***characterized in that** said use of said consortium of micro-organisms of the rhizosphere further obtains in combination:*
i) reduction of nitrates accumulated in plants,
ii) increase of the amount of anti-oxidant substances present in the plants; and
iii) increase of the protein content of the plants.

2. Use according to Claim 1, **characterized in that** said consortium comprises at least two between mycorrhizal fungi, actinomycetes, bacteria of the rhizosphere, saprophytic fungi, and micromycetes.

3. Use according to Claim 2, **characterized in that** said mycorrhizal fungi are selected from among fungi of the genera *Glomus, Acaulospora, Gigaspora* and *Scutellospora,* preferably said mycorrhizal fungi are selected from among fungi of the species *Glomus coronatum, G. celedonium, G. intreradices, G. mosseae, G. viscosum, G. glomaceae, G. fasciculatum, G. claroideum, G. etunicatum, G. epigaeum, G. lamellosum, G. monosporum, Acalospora longula, A. laevis, Gigaspora ramisporophora, G. gigantea, G. rosea, G. calospora,* and *Scutellospora calospora.*

4. Use according to Claim 2, **characterized in that** said actinomycetes are selected among actinomycetes of the genus *Streptomyces spp.,* preferably said actinomycetes are selected among *Streptomyces griseus* and *S. avernichilis.*

5. Use according to Claim 2, **characterized in that** said bacteria of the rhizosphere are selected from among bacteria of the genus *Pseudomonas, Bacillus, Enterobacteriaceae, Paenibacillus, Alcanivorax, Candida, Rhodococcus, Acitenobacter, Mycobaterium, Serratia,* and *Agrobacterium,* preferably said bacteria of the rhizosphere are selected from among bacteria of the species *Pseudomonas borealis, P. fluorescens, P. synxantha, P. corrugata, P. aeroginosa, P. cloraraphis, P. trivelis, P. favisporuns, A. vanetianus, R. rythropolys, A. calcoacetiucus, A. radioresistens, M. thermoautotrophium, S. marcescens, A. radiobacter, B. substilis, B. magaterium, B. polymyxa,* and *B. licheniformis.*

6. Use according to Claim 2, **characterized in that** said saprophytic fungi are selected from among fungi of the genera *Trichoderma, Coniothyrium, Beauveria* and *Gliocladium,* preferably said saprophytic fungi are selected from among fungi of the species *T. harttianum, T. viridae, T. aureoviridae, T. atroviridae, T. koningii, T. virens,* and *T. Hypocrea schweinitzii an., C. minitans, G. calenulatum, B. bassiana.*

7. Use according to Claim 2, **characterized in that** said micromycetes are selected from among fungi of the genera *Mortierella, Aspergillum, Sclerotium, Ulocladium, Arthrobotrys* and *Mucor,* preferably said micromycetes are selected from among micromycetes of the species *M. isabellina, A. niger, U. oudemansii,* and *A. oligospora.*

8. Use according to any one of the preceding claims, **characterized in that** said mycorrhizal fungi are present in an amount of not less than 6 wt% with respect to the total weight of the product, preferably between 10 wt% and 20 wt%.

9. Use according to any one of the preceding claims, **characterized in that** said bacteria of the rhizosphere are present in an amount of not less than 1x10⁶ CFU/g, preferably not less than 5x10⁶ CFU/g, even more preferably from 5 to 10x10⁶ CFU/g, of dry weight with respect to the total weight of the product.

10. Use according to any one of the preceding claims, **characterized in that** said saprophytic fungi and micromycetes are present in an amount of not less than 1x10⁶ CFU/g, preferably not less than 5x10⁶ CFU/g, even more preferably from 5 to 10x10⁶ CFU/g, of dry weight with respect to the total weight of the product.

11. Use according to any one of the preceding claims, **characterized in that** said actinomycetes are present in an amount of not less than 1x10⁶ CFU/g, preferably not less than 5x10⁶ CFU/g, even more preferably from 5 to 10x10⁶ CFU/g, of dry weight with respect to the total weight of the product.

12. Use according to any one of the preceding claims, **characterized in that** said micromycetes are present in an amount of not less than 1x10⁶ CFU/g, preferably not less than 5x10⁶ CFU/g, even more preferably from 5 to 10x10⁶ CFU/g, of dry weight with respect to the total weight of the product.

13. Use according to claim 1, **characterized in that** said mycotoxins are aflatoxins, trichothecenes, ergotamine.

14. Use according to any one of the preceding claims, **characterized in that** said consortium is i) used in the treatment of seeds, ii) mixed in loam and peat for seeding, or iii) spread directly in field, preferably prior to sowing or to transplantation.

## Patentansprüche

1. Verwendung eines Mikroorganismenkonsortiums der Rhizosphäre beim Anbau von Pflanzen zur menschlichen oder tierischen Ernährung zum Erhalten einer Absenkung von in Pflanzen angereicherten schädlichen Substanzen, wobei es sich bei den schädlichen Substanzen um Mycotoxine handelt, **dadurch gekennzeichnet, dass** die Verwendung des Mikroorganismenkonsortiums der Rhizosphäre ferner in Kombination
i) eine Absenkung von in Pflanzen akkumulierten Nitraten,
ii) eine Erhöhung der Menge in den Pflanzen vorhandener antioxidativer Substanzen,
iii) eine Erhöhung des Proteingehalts der Pflanzen
erhält.

2. Verwendung gemäß Anspruch 1, **dadurch gekennzeichnet, dass** das Konsortium mindesten zwei aus Mykorrhizapilzen, Actinomyceten, Bakterien der Rhizosphäre, saprophytischen Pilzen und Mikromyceten umfasst.

3. Verwendung gemäß Anspruch 2, **dadurch gekennzeichnet, dass** die Mykorrhizapilze aus Pilzen der Gattungen *Glomus, Acaulaspora, Gigaspora* und *Scutellospora* ausgewählt sind, vorzugsweise sind die Mykorrhizapilze aus Pilzen der Arten *Glomus coronatum, G. celedonium, G. intreradices, G.* mosseae, *G. viscosum, G. glomaceae, G. fasciculatum, G. claroideum, G. etunicatum, G. epigaeum, G. lamellosum, G. monosporum, Acalospora longula, A. laevis, Gigaspora ramisporophora, G. gigantea, G. rosea, G. calospora* und *Scutellospora calospora* ausgewählt.

4. Verwendung gemäß Anspruch 2, **dadurch gekennzeichnet, dass** die Actinomyceten aus Actinomyceten der Gattung *Streptomyces spp.* ausgewählt sind, vorzugsweise sind die Actinomyceten aus *Streptomyces griseus* und *S. avernichilis* ausgewählt.

5. Verwendung gemäß Anspruch 2, **dadurch gekennzeichnet, dass** die Bakterien der Rhizosphäre aus Bakterien der Gattung *Pseudomonas, Bacillus, Enterobacteriaceae, Paenibacillus, Alcanivorax, Candida, Rhodococcus, Acitenobacter, Mycobaterium, Serratia* and *Agrobacterium* ausgewählt sind, vorzugsweise sind die Bakterien der Rhizosphäre aus Bakterien der Arten *Pseudomonas borealis, P. fluorescens, P. synxantha, P. corrugata, P. aeroginosa, P. cloraraphis, P. trivelis, P. favisporuns, A. vanetianus, R. rythropolys, A. calcoacetiucus, A. radioresistens, M. thermoautotrophium, S. marcescens, A. radiobacter, B. substilis, B. magaterium, B. polymyxa* und *B. licheniformis* ausgewählt.

6. Verwendung gemäß Anspruch 2, **dadurch gekennzeichnet, dass** die saprophytischen Pilze aus Pilzen der Gattungen *Trichoderma, Coniothyrium, Beauveria* und *Gliocladium* ausgewählt sind, vorzugsweise sind die saprophytischen Pilze aus Pilzen der Arten *T. harttianum, T. viridae, T. aureoviridae, T. atroviridae, T. koningii, T. virens, and T. Hypocrea schweinitzii an., C. minitans, G. calenulatum, B. bassiana* ausgewählt.

7. Verwendung gemäß Anspruch 2, **dadurch gekennzeichnet, dass** die Mikromyceten aus Pilzen der Gattungen *Mortierella, Aspergillum, Sclerotium, Ulocladium, Arthrobotrys* und *Mucor* ausgewählt sind, vorzugsweise sind die Mikromyceten aus Mikromyceten der Arten *M. isabellina, A. niger, U. oudemansii* und *A. oligospora* ausgewählt.

8. Verwendung gemäß einem der vorhergehenden Ansprüche, **dadurch gekennzeichnet, dass** die Mykorrhizapilze in einer Menge von nicht weniger als 6 Gew.-% in Bezug auf das Gesamtgewicht des Produkts, vorzugsweise zwischen 10 Gew.-% und 20 Gew.-%, vorhanden sind.

9. Verwendung gemäß einem der vorhergehenden Ansprüche, **dadurch gekennzeichnet, dass** die Bakterien der Rhizosphäre in einer Menge von nicht weniger als 1 x 10⁶ CFU/g, vorzugsweise nicht weniger als 5 x 10⁶ CFU/g, noch stärker bevorzugt 5 bis 10 x 10⁶ CFU/g Trockengewicht in Bezug auf das Gesamtgewicht des Produkts vorhanden sind.

10. Verwendung gemäß einem der vorhergehenden Ansprüche, **dadurch gekennzeichnet, dass** die saprophytischen Pilze und Mikromyceten in einer Menge von nicht weniger als 1 x 10⁶ CFU/g, vorzugsweise nicht weniger als 5 x 10⁶ CFU/g, noch stärker bevorzugt 5 bis 10 x 10⁶ CFU/g Trockengewicht in Bezug auf das Gesamtgewicht des Produkts vorhanden sind.

11. Verwendung gemäß einem der vorhergehenden Ansprüche, **dadurch gekennzeichnet, dass** die Actinomyceten in einer Menge von nicht weniger als 1 x 10⁶ CFU/g, vorzugsweise nicht weniger als 5 x 10⁶ CFU/g, noch stärker bevorzugt 5 bis 10 x 10⁶ CFU/g Trockengewicht in Bezug auf das Gesamtgewicht des Produkts vorhanden sind.

12. Verwendung gemäß einem der vorhergehenden Ansprüche, **dadurch gekennzeichnet, dass** die Mikromyceten in einer Menge von nicht weniger als 1 x 10⁶ CFU/g, vorzugsweise nicht weniger als 5 x 10⁶ CFU/g, noch stärker bevorzugt 5 bis 10 x 10⁶ CFU/g Trockengewicht in Bezug auf das Gesamtgewicht des Produkts vorhanden sind.

13. Verwendung gemäß Anspruch 1, **dadurch gekennzeichnet, dass** es sich bei den Mycotoxinen um Aflatoxine, Trichothecene, Ergotamin handelt.

14. Verwendung gemäß einem der vorhergehenden Ansprüche, **dadurch gekennzeichnet, dass** das Konsortium i) bei der Behandlung von Saatgut verwendet wird, ii) in Lehm und Torf für das Ansäen gemischt wird oder iii) direkt im Feld verteilt wird, vorzugweise vor Aussaat oder Verpflanzung.

## Revendications

1. Utilisation d'un consortium de micro-organismes de la rhizosphère dans la culture de plantes destinées à l'alimentation humaine ou animale, afin d'obtenir
une réduction des substances nocives accumulées dans les plantes, où lesdites substances nocives sont des mycotoxines,
**caractérisée en ce que** ladite utilisation dudit consortium de micro-organismes de la rhizosphère permet en outre d'obtenir, en combinaison :
i) une réduction des nitrates accumulés dans les plantes,
ii) une augmentation de la quantité de substances anti-oxydantes présentes dans les plantes ; et
iii) une augmentation de la teneur en protéines des plantes.

2. Utilisation selon la revendication 1, **caractérisée en ce que** ledit consortium comprend au moins deux éléments parmi des champignons mycorhiziens, des actinomycètes, des bactéries de la rhizosphère, des champignons saprophytes, et des micromycètes.

3. Utilisation selon la revendication 2, **caractérisée en ce que** lesdits champignons mycorhiziens sont sélectionnés parmi des champignons du genre *Glomus, Acaulospora, Gigaspora* et *Scutellospora,* lesdits champignons mycorhiziens étant de préférence sélectionnés parmi des champignons de l'espèce *Glomus coronatum, G. caledonium, G. intraradices, G. mosseae, G. viscosum, G. glomaceae, G. fasciculatum, G. claroideum, G. etunicatum, G. epigaeum, G. lamellosum, G. monosporum, Acaulospora longula, A. laevis, Gigaspora ramisporophora, G. gigantea, G. rosea, G. calospora,* et *Scutellospora calospora.*

4. Utilisation selon la revendication 2, **caractérisée en ce que** lesdits actinomycètes sont sélectionnés parmi des actinomycètes du genre espèce *Streptomyces,* lesdits actinomycètes étant de préférence sélectionnés parmi *Streptomyces griseus* et *S. avernichilis.*

5. Utilisation selon la revendication 2, **caractérisée en ce que** lesdites bactéries de la rhizosphère sont sélectionnées parmi des bactéries du genre *Pseudomonas, Bacillus, Enterobacteriaceae, Paenibacillus, Alcanivorax, Candida, Rhodococcus, Acinetobacter, Mycobacterium, Serratia,* et *Agrobacterium,* lesdites bactéries de la rhizosphère étant de préférence sélectionnées parmi des bactéries de l'espèce *Pseudomonas borealis, P. fluorescens, P. synxantha, P. corrugata, P. aeruginosa, P. cloraraphis, P. trivialis, P. favisporuns, A. vanetianus, R. erythropolis, A. calcoaceticus, A. radioresistens, M. thermoautotrophium, S. marcescens, A. radiobacter, B. subtilis, B. megaterium, B. polymyxa,* et *B. licheniformis.*

6. Utilisation selon la revendication 2, **caractérisée en ce que** lesdits champignons saprophytes sont sélectionnés parmi des champignons du genre *Trichoderma, Coniothyrium, Beauveria* et *Gliocladium,* lesdits champignons saprophytes étant de préférence sélectionnés parmi des champignons de l'espèce *T. harzianum, T. viridae, T. aureoviridae, T. atroviridae, T. koningii, T. virens,* et *T. Hypocrea schweinitzii an., C. minitans, G. catenulatum, B. bassiana.*

7. Utilisation selon la revendication 2, **caractérisée en ce que** lesdits micromycètes sont sélectionnés parmi des champignons du genre *Mortierella, Aspergillum, Sclerotium, Ulocladium, Arthrobotrys* et *Mucor,* lesdits micromycètes étant de préférence sélectionnés parmi des micromycètes de l'espèce *M. isabellina, A. niger, U. oudemansii,* et *A. oligospora.*

8. Utilisation selon l'une quelconque des revendications précédentes, **caractérisée en ce que** lesdits champignons mycorhiziens sont présents en une quantité minimale de 6 % pt par rapport au poids total du produit, de préférence entre 10 % pt et 20 % pt.

9. Utilisation selon l'une quelconque des revendications précédentes, **caractérisée en ce que** lesdites bactéries de la rhizosphère sont présentes en une quantité minimale de 1 x 10⁶ CFU/g, de préférence une quantité minimale de 5 x 10⁶ CFU/g, de manière encore davantage préférée de 5 à 10 x 10⁶ CFU/g, de poids sec par rapport au poids total du produit.

10. Utilisation selon l'une quelconque des revendications précédentes, **caractérisée en ce que** lesdits champignons saprophytes et micromycètes sont présents en une quantité minimale de 1 x 10⁶ CFU/g, de préférence une quantité minimale de 5 x 10⁶ CFU/g, de manière encore davantage préférée de 5 à 10 x 10⁶ CFU/g, de poids sec par rapport au poids total du produit.

11. Utilisation selon l'une quelconque des revendications précédentes, **caractérisée en ce que** lesdits actinomycètes sont présents en une quantité minimale de 1 x 10⁶ CFU/g, de préférence une quantité minimale de 5 x 10⁶ CFU/g, de manière encore davantage préférée de 5 à 10 x 10⁶ CFU/g, de poids sec par rapport au poids total du produit.

12. Utilisation selon l'une quelconque des revendications précédentes, **caractérisée en ce que** lesdits micromycètes sont présents en une quantité minimale de 1 x 10⁶ CFU/g, de préférence une quantité minimale de 5 x 10⁶ CFU/g, de manière encore davantage préférée de 5 à 10 x 10⁶ CFU/g, de poids sec par rapport au poids total du produit.

13. Utilisation selon la revendication 1, **caractérisée en ce que** lesdites mycotoxines sont des aflatoxines, des trichothécènes, l'ergotamine.

14. Utilisation selon l'une quelconque des revendications précédentes, **caractérisée en ce que** ledit consortium est i) utilisé dans le traitement de graines, ii) mélangé au terreau et à la tourbe pour l'ensemencement, ou iii) est directement pulvérisé en champ, de préférence avant le semis ou la transplantation.
